# EUROPÄISCHE PATENTANMELDUNG

(11) **EP 2 238 977 A2**
(43) Veröffentlichungstag der Anmeldung: **13.10.2010**
(21) Anmeldenummer: 10004152.4
(22) Anmeldetag: 21.07.2006
(51) Int. Cl.: A61K 9/70, A61K 47/36, A61K 47/12, A61K 31/17, A61K 47/18, A61K 47/26, A61P 17/00

(54) **Hautpflegeprodukte**

(30) Priorität: 28.09.2005 DE 202005015309 U; 28.09.2005 US 721170 P; 23.03.2006 DE 202006004676 U; 23.03.2006 US 785391 P
(62) Teilanmeldung aus: 06777902.5
(71) Anmelder: Neubourg Skin Care GmbH & Co. KG, 48268 Greven (DE)
(72) Erfinder: Neubourg, Thomas, 59368 Werne (DE)
(74) Vertreter: Maiwald Patentanwalts GmbH

(57) **Zusammenfassung**

Tages- und Nachthautpflegeset, umfassend eine Tageshautpflegezusammensetzung und eine Nachthautpflegezusammensetzung, wobei die Tageshautpflegezusammensetzung eine Schaumhautcreme ist, die mindestens eine freie Fettsäure und mindestens zwei Emulgatoren umfasst, wobei die Nachthautpflegezusammensetzung einen zusätzlichen Wirkstoff oder einen höheren Gew.-%-Anteil an Wirkstoff oder einen anderen Wirkstoff als die Tageshautpflegezusammensetzung umfasst, wobei der Wirkstoff aus der Gruppe der Lipidstoffe und der hydratisierenden Stoffe oder Mischungen davon ausgewählt ist.

## Beschreibung

Die Erfindung betrifft Hautpflegeprodukte insbesondere zur Behandlung und Pflege trockener Hautzustände und chronischer Dermatosen. Die Hautpflegeprodukte sind geeignet für Dermatosen umfassend atopische Dermatitis, das allergische Kontaktekzem, das irritative Kontaktekzem, Psoriasis und sind insbesondere geeignet zur Behandlung von trockener Haut und ihren Begleiterscheinungen wie Entzündungen, Geschwüre etc., die bei Diabetes Mellitus auftreten, dem diabetischen Fußsyndrom.

Ein wesentliches Charakteristikum der Haut beim diabetischen Fußsyndrom (DFS) ist ihre Trockenheit mit der Neigung zur Fissuren- und Rhagadenbildung sowie der Ausprägung von Hyperkeratosen an druckexponierten Stellen.

Beim Diabetiker geht man heute davon aus, dass im Rahmen der autonomen Neuropathie die Sekretion der Schweißdrüsen an den Fußsohlen (zum Teil auch an den Handflächen) reduziert ist. Der lückenhafte Feuchtigkeitsfilm begünstigt direkt und indirekt die Hautaustrocknung durch Abnahme der Verdunstungskühle. Die Zusammenhänge lassen sich wie folgt darstellen. Die autonome Neuropathie führt einerseits zu reduzierter Schweißproduktion und andererseits zur Eröffnung arteriovenöser Shunts (Autosympathektomie), wobei die reduzierte Schweißproduktion zu einem lückenhaften Feuchtigkeitsfilm und zur Abnahme der Verdunstungskühle führt. Diese wiederum führt zusammen mit der Eröffnung arteriovenöser Shunts zur Überwärmung, die zusammen mit dem lückenhaften Flüssigkeitsfilm zur Hautaustrocknung an den Fußsohlen und Handflächen führen. Es hat sich gezeigt, dass die Trockenheit der Diabetikerhaut and den Fußsohlen und Handflächen primär nicht auf Fett sondern auf Feuchtigkeitsmangel basiert.

Es ist Aufgabe der Erfindung, Hautpflegeprodukte herzustellen, die sich für die Behandlung des diabetischen Fußsyndroms aber auch für die Behandlung von Dermatosen wie atopische Dermatitis, das allergische Kontaktekzem, das irritative Kontaktekzem, Psoriasis und insbesondere trockene Haut und ihre Begleiterscheinungen wie Entzündungen, Geschwüre etc., die bei Diabetes Mellitus auftreten, dem diabetischen Fußsyndrom, als auch für die Profilaxe eignen.

Die Aufgabe wird gelöst durch die in den Ansprüchen und der folgenden Beschreibung im Detail beschriebenen Formulierungen und Hautpflegesets.

Die Erfindung betrifft in einem Aspekt Formulierungen, umfassend eine wässrige Emulsion und Treibgas, für eine Schaumhautcreme, **gekennzeichnet dadurch, dass** die Emulsion Harnstoff und Hyaluronsäure, mindestens eine freie Fettsäure und mindestens zwei Emulgatoren umfasst, wobei die Hyaluronsäure ein Molekulargewicht von mindestens 1 x 10⁵ Da aufweist.

Die Erfindung betrifft in einem weiteren Aspekt Formulierungen, umfassend eine wässrige Emulsion und Treibgas, für eine Schaumhautcreme, **gekennzeichnet dadurch, dass** die Emulsion Harnstoff und ein Kohlenhydratgemisch, welches aus natürlich vorkommenden Kohlenhydraten gewonnen wird, mindestens eine freie Fettsäure und mindestens zwei Emulgatoren umfasst.

Die Erfindung betrifft in einem weiteren Aspekt Formulierungen, umfassend eine wässrige Emulsion und Treibgas für eine Schaumhautcreme, **dadurch gekennzeichnet, dass** die Emulsion mindestens eine freie Fettsäure und mindestens zwei Emulgatoren umfasst und mit Hilfe von Kaliumhydroxid auf einen pH-Wert von 5,5 bis 8 eingestellt ist.

Die Erfindung betrifft in einem weiteren Aspekt Tages- und Nachthautpflegesets, umfassend eine Tageshautpflegezusammensetzung und eine Nachthautpflegezusammensetzung, wobei die Tageshautpflegezusammensetzung eine Schaumhautcreme ist, die mindestens eine freie Fettsäure und mindestens zwei Emulgatoren umfasst, wobei die Nachthautpflegezusammensetzung einen zusätzlichen Wirkstoff oder einen höheren Gew.-%-Anteil an Wirkstoff oder einen anderen Wirkstoff als die Tageshautpflegezusammensetzung umfasst, wobei der Wirkstoff aus der Gruppe der Lipidstoffe und der hydratisierenden Stoffe oder Mischungen davon ausgewählt ist.

Gemäß einem Aspekt betrifft die Erfindung die Verwendung einer Formulierung oder eines Tages- und Nachtpflegesets gemäß der Erfindung zur Behandlung von Dermatosen, sowie Verfahren zur Behandlung von Dermatosen, bei welchen eine Formulierung gemäß der Erfindung auf den zu behandelnden Hautbereich aufgetragen wird. Ferner betrifft die Erfindung gemäß einem Aspekt Verfahren zur Behandlung von Dermatosen, bei welchem nach dem Aufstehen für die Tagespflege eine Tageshautpflegezusammensetzung und vor dem Schlafengehen für die Nachtpflege eine Nachthautpflegezusammensetzung gemäß der Erfindung auf die zu behandelnde Haut aufgetragen wird. Bei den Dermatosen handelt es sich z.B. um trockene Hautzustände, chronische Dermatosen, atopische Dermatitis, das allergische Kontaktekzem, das irritative Kontaktekzem, Psoriasis und das diabetische Fußsyndrom.

Im Folgenden werden die einzelnen Aspekte der Erfindung im Detail beschrieben. Diese einzelnen Aspekte der Erfindung stehen für sich, können aber auch vorteilhaft miteinander kombiniert werden. Die einzelnen beschriebenen Merkmale sind daher jeweils frei mit allen anderen Merkmalen, die im Rahmen dieser Erfindung beschrieben werden, kombinierbar.

In einem Aspekt betrifft die Erfindung Schaumcremes.

Schaumcremes sind Aerosole die aus einer Emulsion mit Hilfe von Treibgas durch Versprühen aus einem geeigneten Behältnis/Darreichungsform gebildet werden.

Schaumcreme-Formulierungen enthalten daher im Allgemeinen eine Emulsion und Treibgas. Im Rahmen der vorliegenden Erfindung bezieht sich der Ausdruck "Formulierung" auf alle Bestandteile, die nötig sind, um die Schaumcreme zu bilden, d.h. die Emulsion und das Treibgas, währen der Ausdruck "Emulsion" sich auf alle Bestandteile außer dem Treibgas bezieht.

Wie bereits erwähnt, zählen Schäume zu Aerosolen und sind komplizierte, physikalisch-chemische Gebilde, die einer besonderen Abstimmung der den Schaum bildenden Komponenten bedürfen. Die Besonderheit der Schaumcreme der vorliegenden Erfindung ist, dass diese durch ihre stabile zweiphasige polydisperse Struktur bei der Applikation auf die Haut eine Netzstruktur, vergleichbar mit einer Membran, bildet. Insbesondere der Fettsäurebestandteil bildet feinmaschige netzartige Kristallisate, die wie ein Sieb mit einem kleinen Benetzungswinkel den Kontakt von Wasser mit der Haut unterbinden. Andererseits ist die Haut zwischen den Maschen praktisch unverändert und kann daher ungehindert einen Gasaustausch mit der Umgebung weiterhin durchführen. Es kommt also praktisch zu keiner Behinderung der Perspiratio insensibilis, zu keinem Wärmestau und durch den durch den Schaum automatisch vorgegebenen dünnen Auftrag auch zu keiner Veränderung der Griffigkeit. Diese Eigenschaften erlauben eine unveränderte Hautatmung bei gleichzeitiger Schutzfunktion.

Die erfindungsgemäße Schaumhautcreme wird erzeugt durch Versprühen einer Formulierung, die eine wässrige Emulsion und Treibgas enthält, aus einem unter Druck stehenden Behältnis. Als Treibgas können alle pharmazeutisch unbedenklichen Treibgase verwendet werden, die sich zur Erzeugung von Cremeschäumen eignen.

Im Rahmen der vorliegenden Erfindung enthält die wässrige Emulsion alle Bestandteile der Formulierung außer dem Treibgas.

Gemäß einem Aspekt enthält die Schaumcreme Harnstoff und Hyaluronsäure. Insbesondere enthält diese erfindungsgemäße Schaumcreme Harnstoff und hochmolekulare Hyaluronsäure mit einem Molekulargewicht M von mindestens 1 x 10⁵ Da, von mindestens 5 x 10⁵ Da, von mindestens 1 x 10⁶ Da oder von mindestens 1,5 x 10⁶ Da. Gemäß eines Aspektes der Erfindung wird Hyaluronsäure von etwa 1,7 x 10⁶ Da eingesetzt. Im Rahmen dieser Erfindung bedeutet Hyaluronsäure entweder die freie Hyaluronsäure oder ein entsprechendes pharmazeutisch akzeptables Derivat, insbesondere ein pharmazeutisch akzeptables Salz. Mengen- und Größenangaben beziehen sich im Rahmen dieser Erfindung jedoch immer auf die freie Hyaluronsäure.

Der Harnstoff bewirkt eine Erhöhung der Wasserbindungsfähigkeit der Hornschicht sowohl inter- als auch intrazellulär. Sein geringes Molekulargewicht und die daraus resultierenden starken osmotischen Eigenschaften sowie seine Polarität und die daraus resultierenden hygroskopischen Eigenschaften verleihen dem Harnstoff seine Wirksamkeit. In nativer Form liegt Harnstoff als bipolares Molekül vor, an dessen positivem bzw. negativem Pol sich bevorzugt Wassermoleküle über Wasserstoffbrückenbindung anlagern und somit die Wasserbindekapazität des Harnstoffes begründen.

Hyaluronsäure ist ein natürliches, körpereigenes Polymer aus Kohlehydraten und stark hygroskopisch. Aufgrund seiner physikalisch-chemischen Struktur bildet Hyaluronsäure ein Netzwerk von Polysacchariden, das in der Lage ist Wasser zu binden und die Elastizität der Epidermis zu verbessern. Insbesondere die hochmolekulare Hyaluronsäure ist in der Lage, solche Netzwerke auszubilden und durch ihre physikalischen Eigenschaften innerhalb der erfindungsgemäßen Schaumcreme zu wirken. Insbesondere hat sich im Rahmen dieser Erfindung gezeigt, dass sich die Hyaluronsäure stabilisierend auf die Schaumstruktur auswirkt und damit für die Ausbildung der Netzstruktur auf der Haut und die damit verbundene Schutzfunktion sorgt.

Gemäß dieses Aspektes versorgen die erfindungsgemäßen Cremeschäume die Haut, insbesondere die besonders strapazierte und trockene Haut bei Diabetikern, mit genügend Feuchtigkeit, ohne die Poren zu verstopfen und somit ohne zu einer weiteren Reduktion der Schweißbildung zu führen. Ferner verhindern die erfindungsgemäßen Cremeschäume eine Überladung der Haut mit fettenden Stoffen und das Hervorrufen eines unangenehm fettigen Gefühls. Die erfindungsgemäßen Cremeschäume ziehen schnell ein und hinterlassen ein angenehmes Hautgefühl ohne zu fetten. Diese Cremeschäume eignen sich damit besonders für Tagespflegeprodukte.

Die wässrige Emulsion umfasst gemäß dieses Aspektes der Erfindung Harnstoff, Hyaluronsäure, mindestens eine freie Fettsäure und mindestens zwei Emulgatoren.

Im Folgenden wird nun die Zusammensetzung der Emulsion näher beschrieben.

Gemäß dieser Ausführungsform umfasst die Schaumcreme Harnstoff und hochmolekulare Hyaluronsäure. Die Hyaluronsäure weist mindestens ein Molekulargewicht von 1 x 10⁵ Da oder von mindestens 5 x 10⁵ Da oder von mindestens 1 x 10⁶ Da und insbesondere von mindestens 1,5 x 10⁶ Da auf. Gemäß einer Ausführungsform wird Hyaluronsäure von etwa 1,7 x 10⁶ Da eingesetzt.

Gemäß einer Ausführungsform umfasst die Emulsion 3 - 30 Gew.-% Harnstoff, 4 - 30 Gew.-% Harnstoff, 5 -20 Gew.-% Harnstoff oder 5 -17 Gew.-% Harnstoff. Bevorzugte Ausführungsformen enthalten mehr als 5 Gew.-% Harnstoff, wie etwa 5,5 - 25 Gew.-% oder 5,5, 11 oder 16,5 Gew.-% Harnstoff.

Die Emulsion umfasst ferner gemäß einer Ausführungsform 0,01 - 5 Gew.-% Hyaluronsäure (basierend auf dem Gewicht der freien Säure), 0,01 - 3 Gew.-%, oder 0,1 - 1 Gew.-% Hyaluronsäure (basierend auf dem Gewicht der freien Säure). Bevorzugte Ausführungsformen enthalten weniger als 1 Gew.-% Hyaluronsäure, oder weniger als 0,5 Gew.-% Hyaluronsäure, wie etwa 0,01 - 0,9 Gew.-%, 0,01 - 0,5 Gew.-% oder 0,2 Gew.-% Hyaluronsäure.

Gemäß eines anderen Aspektes der Erfindung enthält die Schaumcreme Harnstoff und ein Kohlenhydrat-Gemisch, welches aus natürlich vorkommenden Kohlenhydraten gewonnen wird. Gemäß einer Ausführungsform der Erfindung entspricht das Kohlenhydrat-Gemisch in seiner Zusammensetzung der in Humanhaut enthaltenen Kohlenhydraten. Dieses Kohlenhydrat-Gemisch wird beispielsweise in Form einer wässrigen Lösung mit 50 - 55 Gew.-% Trockenrückstand eingesetzt, wie es beispielsweise unter der Handelsbezeichnung Pentavitin® von der Firma Pentapharm erhältlich ist. Es handelt sich hierbei um ein Produkt, welches durch vorsichtig durchgeführte Isomerisation von aus Pflanzen gewonnener D-Glucose erhalten wird. Solche Kohlenhydrat-Gemische sind dafür geeignet, Wasser in der Haut wirkungsvoll und lang anhaltend zu binden. Solche Kohlenhydrat-Gemische nennt man auch Feuchtigkeitsmagnete. Diese tragen in den erfindungsgemäßen Formulierungen dazu bei, die Feuchtigkeit, die beispielsweise durch Harnstoff und/oder Hyaluronsäure gespendet wird, in der Haut zu binden.

Gemäß dieses Aspektes versorgen die erfindungsgemäßen Cremeschäume die Haut, insbesondere die strapazierte und trockene Haut bei Diabetikern, mit genügend Feuchtigkeit, ohne die Poren zu verstopfen, und sind zusätzlich in der Lage, die Feuchtigkeit lang anhaltend zu binden. Auch hier verhindern die erfindungsgemäßen Cremeschäume eine Überladung der Haut mit fettenden Stoffen und das Hervorrufen eines unangenehmen fettigen Gefühls. Die Cremeschäume ziehen schnell ein und hinterlassen ein angenehmes Hautgefühl ohne zu fetten. Auch diese Cremeschaumformulierungen eignen sich gut für die Tages- und Nachtpflege.

Im Folgenden wird nun die Zusammensetzung der Emulsion näher beschrieben.

Gemäß dieser Ausführungsform umfasst die Schaumcreme Harnstoff und das Kohlenhydrat-Gemisch. Gemäß einer Ausführungsform ist das Kohlenhydrat-Gemisch aus natürlich vorkommenden Kohlenhydraten gewonnen und entspricht bevorzugt in seiner Zusammensetzung den in der Humanhaut enthaltenen Kohlenhydraten. Das Kohlenhydrat-Gemisch kann in Form einer wässrigen Lösung mit 50 - 55 Gew.-% Trockenrückstand eingesetzt werden, wie es im Handel unter der Bezeichnung Pentavitin® erhältlich ist.

Gemäß einer Ausführungsform umfasst die Emulsion 3 - 30 Gew.-% Harnstoff, 4 - 30 Gew.-% Harnstoff, 5 - 20 Gew.-% Harnstoff oder 5 - 17 Gew.-% Harnstoff. Bevorzugte Ausführungsformen enthalten mehr als 5 Gew.-% Harnstoff, wie etwa 5,5 - 25 Gew.-% Harnstoff, wie etwa 5,5, 11 oder 16,5 Gew.-% Harnstoff.

Die Emulsion umfasst ferner gemäß einer Ausführungsform 0,01 - 5 Gew.-% der wässrigen Lösung des Kohlenhydratgemisches, 0,01 - 3 Gew.-% dieser wässrigen Lösung und bevorzugt etwa 0,6 Gew.-% oder 1,25 Gew.-% dieser wässrigen Lösung.

Gemäß einer weiteren Ausführungsform der Erfindung umfasst die Emulsion Harnstoff, hochmolekulare Hyaluronsäure und das Kohlenhydrat-Gemisch in den Formen und Mengen, wie voranstehend beschrieben. Gemäß einer Ausführungsform enthält die Emulsion 5-17 Gew.-% Harnstoff, oder mehr als 5 Gew.-% Harnstoff, wie etwa 5,5 bis 25 Gew.-% Harnstoff, 0,01 - 1 Gew.-% Hyaluronsäure oder weniger als 1 Gew.-% Hyaluronsäure, wie etwa 0,001 - 0,9 Gew.-% oder 0,01 - 0,9 Gew.-% Hyaluronsäure und 0,01 - 2 Gew.-% des Kohlenhydrat-Gemisches. Gemäß einer bevorzugten Ausführungsform enthält die Emulsion 5-17 Gew.-% Harnstoff, etwa 0,2 Gew.-% Hyaluronsäure und etwa 0,6 Gew.-% oder etwa 1,25 Gew.-% des Kohlenhydrat-Gemisches in Form der wässrigen Lösung.

Ein weiterer Aspekt dieser Erfindung betrifft die Schaumstabilität (Stabilität der Schaumstruktur), insbesondere die Lagerstabilität der Formulierung im Druckbehälter bezüglich der resultierenden Schaumstabilität. Wie bereits erwähnt, zählen Schäume zu Aerosolen und sind komplizierte physikalische Gebilde, die gerade durch ihre stabile zweiphasige polydisperse Struktur bei der Applikation auf der Haut eine Netzstruktur bilden, die zu den Vorteilen der Schaumformulierungen beiträgt. Wenn die Schaumstruktur zu schnell kollabiert, kann diese Netzstruktur nicht ausgebildet werden. Gemäß eines Aspektes dieser Erfindung wird die Stabilität der Schaumstruktur, insbesondere die Schaumstabilität nach Lagerung, verbessert.

Es hat sich gezeigt, dass die Stabilität des Schaums bzw. der Schaumstruktur durch die Wahl der zur Einstellung des pH-Wertes verwendeten Base positiv beeinflusst werden kann. Es hat sich gezeigt, dass unter den Basen, wie Aminomethylpropanol (AMP), Natriumhydroxid (NaOH) und Kaliumhydroxid (KOH), die Verwendung von Kaliumhydroxid eine Verbesserung der Schaumstabilität, insbesondere nach Lagerung zwischen 10 und 40 °C bewirkt. Dieser Effekt hat sich gezeigt bei Verwendung einer Basis-Emulsion umfassend freie Fettsäuren und mindestens zwei Emulgatoren sowie in Kombination mit verschiedenen Wirkstoffen. Gemäß dieses Aspektes der Erfindung umfasst die Schaumformulierung eine wässrige Emulsion und Treibgas, wobei die Emulsion mindestens eine freie Fettsäure und mindestens zwei Emulgatoren umfasst und mit Hilfe von Kaliumhydroxid auf einen pH-Wert von 5,5 - 8 eingestellt ist.

Im Rahmen dieser Erfindung betrifft der Ausdruck "freie Fettsäure" eine freie Fettsäure, ein Gemisch aus freien Fettsäuren sowie eine partiell verseifte Fettsäure, bzw. ein Gemisch von partiell verseiften Fettsäuren.

Es hat sich ferner herausgestellt, dass auch die Zugabe von Hyaluronsäure sich stabilisierend auf die Schaumstruktur auswirkt. Insbesondere hat sich gezeigt, dass die Zugabe von 0,2 Gew.-% Hyaluronsäure sich besonders stabilisierend auswirkt. Gemäß dieses Aspektes der Erfindung umfasst die Emulsion mindestens eine freie Fettsäure, mindestens zwei Emulgatoren und etwa 0,2 Gew.-% Hyaluronsäure und ist mit Hilfe von Kaliumhydroxid auf einen pH-Wert von 5,5 bis 8 eingestellt. Gemäß eines Aspektes dieser Erfindung ist die Emulsion mit Kaliumhydroxid auf einen pH-Wert von 6,5 bis 7,5 eingestellt.

Dieser beschriebene Stabilitätszugewinn durch die pH-Wert-Einstellung mit Hilfe von Kaliumhydroxid sowie auch die Stabilitätserhöhung durch die Zugabe von Hyaluronsäure kann gut mit allen anderen Aspekten der Erfindung, die hier beschrieben werden, kombiniert werden.

Im Folgenden wird nun die Emulsionsgrundlage der Basis-Emulsion beschrieben, die sich mit allen Aspekten dieser Erfindung gut kombinieren lässt, wie beispielsweise mit den Inhaltsstoffen/Wirkstoffen Harnstoff, Hyaluronsäure und Kohlenhydrat-Gemisch sowie der pH-Wert-Einstellung mit Kaliumhydroxid, wie vorstehend beschrieben, als auch mit den nachfolgend beschriebenen Tages- und Nachthautpflegesets.

Die Emulsion ist bevorzugt eine Öl-in-Wasser-Emulsion (O/W-Emulsion), die beim Entweichen aus dem Ventil des Druckbehälters durch die Expansion des in der inneren Ölphase gelösten Treibgases aufschäumt.

Solche O/W-Emulsionen bestehen aus einer lipophilen Phase I und einer hydrophilen Phase II, die durch Zugabe von Emulgatoren unter geeigneten Mischbedingungen Emulsionen bilden, wobei die äußere Phase die Wasserphase/hydrophile Phase und die innere Phase die Ölphase/lipophile Phase ist. Hierzu wird erfindungsgemäß die lipophile/Öl-Phase oder auch Fettphase aufgeschmolzen oder erwärmt, sowie mit einem geeigneten Emulgator (ein Emulgator oder eine Mischung aus mehreren verschiedenen Emulgatoren) versetzt und unter Rühren zu der ebenfalls erwärmten hydrophilen Phase/Wasserphase, die ebenfalls Emulgator (einen Emulgator oder eine Mischung aus mehreren verschiedenen Emulgatoren) enthält, dosiert. Die Zudosierung der hydrophilen Phase zur lipophilen Phase ist ebenfalls möglich. Die homogenisierte Mischung wird erfindungsgemäß anschließend abgekühlt, und der pH-Wert wird, wenn nötig, eingestellt. Die abgekühlte erhaltene Mischung/Emulsion wird dann unter Zugabe eines Treibgases in eine geeignete Darreichungsform abgefüllt.

Die erfindungsgemäßen Emulsionen enthalten freie Frettsäuren und mindestens zwei Emulgatoren. Bevorzugt werden C₁₂- bis C₂₂-Fettsäuren, insbesondere natürliche C₁₂- bis C₂₀-Fettsäuren, wie etwa Stearinsäure, Palmitinsäure und Myrisitinsäure oder Mischungen hiervon eingesetzt. Besonders bevorzugt ist Stearinsäure. Stearinsäure bedeutet im Rahmen der vorliegenden Erfindung entweder im Wesentlichen reine Stearinsäure oder ein Gemisch höherer Fettsäuren wie Stearinsäure und Palmitinsäure. Die Fettsäuren stellen einen Bestandteil der lipophilen Phase/Fettphase dar. Die erfindungsgemäße Emulsion enthält bevorzugt 1 bis 10 Gew.-% Fettsäuren, mehr bevorzugt 4 bis 7 Gew.-% Fettsäure und besonders bevorzugt 4.5 bis 6 Gew.-% Fettsäuren.

Gemäß einer Ausführungsform der Erfindung enthält die Emulsion ferner ungesättigte und/oder mehrfach ungesättigte Fettsäuren, wie z.B. Omega-6-Fettsäuren, wie sie beispielsweise aus Nachtkerzenöl und Borretschöl bekannt sind. Es können z.B. synthetische Omega-6-Fettsäuren oder auch solche aus Pflanzen gewonnenen Fettsäuren eingesetzt werden. Gemäß eines weiteren Aspektes der Erfindung enthält die Emulsion Nachtkerzenöl.

Die Emulsion umfasst ferner mindestens zwei verschiedene Emulgatoren, die zum einen aus der Gruppe der nichtionischen Emulgatoren, insbesondere solcher auf Fettalkoholbasis und auf Basis von Partialestern von Fettsäuren wie etwa Cetearylalkohol oder Glycerylstearat, zum anderen aus der Gruppe der anionischen Emulgatoren, insbesondere der Alkylsarcosinate, wie Lauryl, Laurolyl oder Cetylsarcosinat ausgewählt sind. Vorzugsweise werden die nichtionischen Emulgatoren bei der Herstellung zunächst der Fettphase und die anionischen Emulgatoren der Wasserphase zugegeben. Die erfindungsgemäße Emulsion enthält bevorzugt 4 bis 15 Gew.-% dieser Emulgatoren. Bevorzugt enthält die Emulsion mindesten drei dieser Emulgatoren, wobei zwei Emulgatoren aus der Gruppe der nichtionischen Emulgatoren ausgewählt sind und ein Emulgator aus der Gruppe der ionischen Emulgatoren ausgewählt ist. Am meisten bevorzugt ist dabei die Kombination von Cetearylalkohol, Glycerylstearat und Laurylsarcosinat. Hierbei wird bevorzugt 1 bis 3 Gew.-% Glycerylstearat, 3 bis 6 Gew.-% Cetearylalkohol und 1 bis 3 Gew.-% Laurylsarcosinat eingesetzt. Weitere mögliche Emulgatoren bzw. stabilisierende Inhaltsstoffe sind Cetearyl Glycosid, Hydroxyethylacrylat, Natriumacryloyldimethyltaurat Copolymer, Polysorbat 60, Squalan, Mischungen dieser und Ähnliches.

In einer bevorzugten Ausführungsform der Erfindung umfasst die Emulsion ferner mindestens einen weiteren Emulgator, der im Rahmen der vorliegenden Erfindung Coemulgator bezeichnet wird. Dieser Coemulgator ist aus der Gruppe der lipophilen Emulgatoren, insbesondere aus der Gruppe bestehend aus Tricetearet-4-phosphat, Olet-3-phosphat sowie weiteren lipophile Emulgatoren auf Basis niedrig ethoxylierter Fettalkohole ausgewählt. Der Coemulgator wird vorzugsweise bei der Herstellung zunächst der Fettphase zugegeben. Bevorzugt werden 0.4 bis 2.5 Gew.-% eingesetzt. Bevorzugt wird Tricetearet-4-phosphat in Mengen von 0.4 bis 2.5 Gew.-% eingesetzt.

Gemäß einer weiteren bevorzugten Ausführungsform enthält die Emulsion ferner rückfettende Stoffe. Die rückfettenden Stoffe werden vorzugsweise aus der Gruppe bestehend aus Decyloleat, Isohexadecan, Stearinsäureglykolester, Kokosfettsäureethanolamid, Maisöl, Erdnussöl, Mandelöl, Sesamöl, Olivenöl, Jojobaöl, Sojaöl, Wollwachsalkohole, Paraffin, mittelkettige Triglyceride, Ölsäureoleylester, weißes Vaselin, Macrogol-Glycerolhydroxistearat, hydriertes Rizinusöl, Rizinusöl communis, Avocadoöl, Weizenkeimöl, Nachtkerzenöl, Sheabutter, Palmitinsäureisopropylester, Cetylpalmitat, Myristinsäuremyristilester und Octyldodecanol ausgewählt. Die erfindungsgemäßen Emulsionen enthalten 0,5 - 6 Gew.-% rückfettende Stoffe, bevorzugt sind 0,5 - 2 Gew.-% rückfettende Stoffe. Weitere Ölkomponenten sind C₁₂ -C₁₃-Alkyllactate und C₁₂ -C₁₅-Alkylbenzoate. Ein zu hoher Gehalt an fettenden Substanzen sollte jedoch gemäß manchen Aspekten der Erfindung vermieden werden. Für die Tagespflege eignen sich fettarme Produkte und für die Nachtpflege fettreiche Produkte. Gemäß einer Ausführungsform ist die Emulsion frei von Paraffin.

Die erfindungsgemäßen Emulsionen können ferner Moisturiser, Konservierungsmittel, reizlindernde Substanzen, Hautpflegemittel und hautwirksame Vitamine sowie siliziumhaltige Substanzen, wie etwa Dimethicon, enthalten.

Als weitere Moisturiser kommen in Betracht Propylenglycol und/oder mehrwertige Alkohole, insbesondere Glycerin. Moisturiser können in Mengen von 1 - 10 Gew.-%., bevorzugt von 2,5 - 8.5 Gew.-% zugegeben werden. Bevorzugt wird Glycerin und Propylenglycol zugegeben.

Als Konservierungsstoffe eignen sich insbesondere Stoffe wie Paraben, Methyldibromoglutaronitril und/oder Phenoxyethanol. Konservierungsstoffe können in Mengen von 0,01 - 1 Gew.-%. zugegeben werden. Bevorzugt werden jedoch keine Konservierungsmittel zugegeben.

Geeignete reizlindernde Substanzen, Hautpflegemittel und hautwirksame Vitamine sind Kamillen-, Calendula-, Hamamelis-, und Teebaumölextrakt, Portulak, Allantoin, Panthenol, und die Vitamine A, E und F. Diese Bestandteile können in Mengen von 0,01 - 2,0 % oder 0,05 - 1 Gew.-% zugegeben werden. Bevorzugt wird Allantoin zugegeben.

Als reizlindernde Substanz wird gemäß eines Aspektes der Erfindung Portulak verwendet. Portulak kann in Mengen von 0,01 bis 2 Gew.-% eingesetzt werden, bevorzugt etwa 0,1 Gew.-%.

Die erfindungsgemäßen Formulierungen weisen ferner einen hautverträglichen pH-Wert auf, wie etwa von 7,0 bis 8,0, 5,5 bis 8 oder 6,5 bis 7,5. Hierfür kann auch 2-Amino-2-methyl-1-propanol (AMP) eingesetzt werden. Gemäß eines Aspektes wird hierfür Kaliumhydroxid, wie voranstehend beschrieben, eingesetzt.

Eine erfindungsgemäße Formulierung enthält eine Emulsion, die 1 - 10 Gew.-% Fettsäuren, 4 - 15 Gew.-% Emulgatoren, 0,4 - 2,5 Gew.-%. Coemulgatoren, 1 - 10 Gew.-% Moisturiser, 0,05 - 1 Gew.-% Hautpflegemittel und 0,5 - 6 Gew.-% rückfettende Stoffe umfasst. Eine weitere erfindungsgemäße Formulierung enthält eine Emulsion, die 3 - 7 Gew.-% Stearinsäure, 4 -10 Gew.-% Emulgatoren, 0,4 - 2 Gew.-% Coemulgatoren, 2,5 - 8,5 Gew.-% Moisturiser, 0,5 - 2 Gew.-% rückfettende Stoffe und 0,05 - 1 Gew.-% Hautpflegemittel umfasst. Bevorzugt werden in dieser Emulsion Glycerylstearat und Cetarylalkohol und Natriumlaurylsarcosinat als Emulgatoren und Tricetearet-4-phosphat als Coemulgator eingesetzt. Als rückfettender Stoff wird bevorzugt Decyloleat und Octyldodecanol als Moisturiser bevorzugt Glycerin und als Hautpflegemittel bevorzugt Allantoin eingesetzt. Der Emulsion kann dann zum Beispiel, wie beschrieben, Harnstoff, Hyaluronsäure und Pentavitin zugegeben werden.

Wasser ist zum Ausgleich auf 100% zugegeben.

Die erfindungsgemäßen Formulierungen werden bevorzugt wie folgt hergestellt:
- Herstellen einer Phase I durch Aufschmelzen bei 70° C - 80° C, bevorzugt bei 75° C einer Mischung, umfassend mindestens eine Fettsäure, mindestens einen nichtionischen Emulgator und mindestens einen Coemulgator, sowie gegebenenfalls die rückfettenden Stoffe,
- gefolgt von einer dosierten Zugabe unter Rühren zu einer auf 70° - 80° C, bevorzugt 75° C temperierten Phase II, umfassend Wasser, Harnstoff, Hyaluronsäure und mindestens einen anionischen Emulgator sowie gegebenenfalls Moisturiser und Hautpflegemittel, um eine homogene Mischung/Emulsion aus Phase I und Phase II herzustellen,
- Abkühlen der Mischung/Emulsion unter Rühren auf eine Temperatur von Raumtemperatur bis 40° C, bevorzugt 30° C bis 40° C und
- wenn nötig, Einstellen des pH-Wertes, bevorzugt auf einen pH-Wert von 5,5 - 8.
- Abfüllen der erhaltenen Mischung/Emulsion in eine geeignete Darreichungsform unter Zugabe eines Treibgases.

Gemäß eines weiteren Aspektes betrifft die Erfindung ein Tages- und Nachthautpflegeset, umfassend eine Tageshautpflegezusammensetzung und eine Nachthautpflegezusammensetzung, wobei die Tageshautpflegezusammensetzung eine Schaumhautcreme ist, die mindestens eine freie Fettsäure und mindestens zwei Emulgatoren umfasst, und wobei die Nachthautpflegezusammensetzung einen zusätzlichen Wirkstoff oder einen höheren Gewichtsprozentanteil an Wirkstoff oder andere Wirkstoffe als die Tageshautpflegezusammensetzung enthält, wobei der Wirkstoff aus der Gruppe der Lipidstoffe und der hydratisierenden Stoffe oder Mischungen davon ausgewählt ist.

Tag und Nacht unterscheiden sich in Bezug auf die Hautpflege durch unterschiedliche Anforderungen an den Anwendungskomfort bzw. den Pflegeanspruch. Unter tags ist es wichtig, dass die Hautpflege wenig fettend ist und wenig bei den Aktivitäten des Tages stört. Zudem ist eine erhöhte Schutzfunktion erwünscht. In der Nacht muss weniger auf den Anwendungskomfort sowie weniger auf die Schutzfunktion Rücksicht genommen werden und es kann mehr auf pflegende und zumeist auch mehr fettende Substanzen zurückgegriffen werden. Die Nachtzeit kann somit wirkungsvoll für die Pflege und Regeneration verwendet werden, während unter tags die Schutzfunktion und der Anwendungskomfort überwiegen. Dadurch kann eine Kombination in Form eines Tages- und Nachthautpflegesets, umfassend eine speziell auf die Anforderungen des Tages ausgerichtete Tageshautpflegezusammensetzung und eine speziell für die Nacht ausgelegte Nachthautpflegezusammensetzung auf diese Bedürfnisse speziell eingehen. Als Tageshautpflegezusammensetzung wird gemäß dieser Erfindung eine Schaumhautcreme verwendet, die zum Beispiel eine der voranstehend beschriebenen Formulierungen sein kann. Gerade die Schaumformulierung bietet einen erhöhten Anwendungskomfort und eine gute Schutzfunktion, die der Tagesanwendung entsprechen. Als Nachthautpflegezusammensetzung kommt ebenfalls eine Schaumhautcreme in Frage, jedoch können hier auch fettigere Salben und Lotionen verwendet werden. Gemäß einer Ausführungsform der Erfindung sind die enthaltenen Wirkstoffe, wie Lipidstoffe und hydratisierende Stoffe der Tageshautpflegezusammensetzung und der Nachthautpflegezusammensetzung aufeinander abgestimmt. Zum einen können beide Hautpflegezusammensetzungen den selben Wirkstoff oder die selbe Wirkstoffkombination enthalten, wobei die Nachthautpflegezusammensetzung zum Beispiel vergleichsweise höhere Anteile an Wirkstoffen als die Tageshautpflegezusammensetzung enthält. Ferner kann auch die Nachthautpflegezusammensetzung einen zusätzlichen Wirkstoff oder andere Wirkstoffe enthalten. Hierbei kommen jegliche Wirkstoffkombinationen in Frage, insbesondere die bereits vorstehend beschriebenen Wirkstoffkombinationen, wie Harnstoff, Hyaluronsäure und das Kohlenhydrat-Gemisch. Hierbei handelt es sich um hydratisierende Stoffe, die Feuchtigkeit zuführen und in der Haut speichern. Ferner können Lipidstoffe verwendet werden, wie beispielsweise Fettsäuren, unter anderem ungesättigte Fettsäuren sowie jegliche rückfettende Stoffe. Gemäß einer Ausführungsform der Erfindung wird Nachtkerzenöl, welches reich an ungesättigten Fettsäuren ist, als Lipidstoff eingesetzt.

Gemäß eines weiteren Aspektes der Erfindung kann unter Umständen auch die oben beschriebene Nachthautpflegezusammensetzung als

Tageshautpflegezusammensetzung eingesetzt werden sowie die oben beschriebene Tageshautpflegezusammensetzung als Nachthautpflegezusammensetzung.

Gemäß eines Aspektes der Erfindung ist die Tageshautpflegezusammensetzung eine Formulierung enthaltend Harnstoff und Hyaluronsäure, wie voranstehend beschrieben und die Nachthautpflegezusammensetzung eine Formulierung enthaltend Harnstoff, Hyaluronsäure und das Kohlenhydrat-Gemisch. Insbesondere kommt hier für den Tageshautcremeschaum eine Formulierung in Frage, die 11 % Harnstoff, 0,2 % Hyaluronsäure enthält und durch die Zugabe von KOH als Base auf einen pH-Wert von etwa 7 eingestellt ist. Diese wird mit einer Nachthautpflege, umfassend eine Formulierung mit 11 Gew.-% Harnstoff, 0,2 Gew.-% Hyaluronsäure sowie 0,6 bzw. 1,25 Gew.-% des Kohlenhydrat-Gemisches in Form von Pentavitin® und mit KOH mit einem pH-Wert von etwa 7 eingestellt, kombiniert.

Gemäß einem weiteren Aspekt ist die Tageshautpflege eine Formulierung, die Harnstoff, bevorzugt 5 bis 17 % Harnstoff oder 5 bis 15 % Harnstoff, enthält, und die Nachthautpflege ist eine Formulierung, die ein Kohlenhydrat-Gemisch und gegebenenfalls Hyaluronsäure, wie in dieser Anmeldung beschrieben, enthält, oder umgekehrt.

Gemäß eines anderen Aspektes ist die Tageshautpflege eine Formulierung, die 16,5 % Harnstoff und 1 % Nachtkerzenöl enthält, wobei diese ebenfalls mit KOH auf einem pH-Wert von etwa 7 eingestellt sein kann. Als Nachthautpflege wird zum Beispiel eine Salbe mit der im Folgenden gezeigten beispielhaften Zusammensetzung eingesetzt.

Das Tages- und Nachthautpflegeset gemäß der vorliegenden Erfindung und wie voranstehend beschrieben, umfassend eine Tageshautpflegezusammensetzung und eine Nachthautpflegezusammensetzung, ist für viele Anwendungen geeignet. Zum Beispiel zur Behandlung und Pflege trockener Hautzustände und chronischer Dermatosen. Aber auch belastete Hautpartien, wie beispielsweise Hände und Füße von Sportlern, können behandelt werden und (weiteren) Hautschäden kann vorgebeugt werden.

| Rohstoff | Handelsbezeichnung | Gew.-%. |
|---|---|---|
| Harnstoff | | 15 |
| Glycerin | | 6 |
| Glyceryl Stearate SE | (Tegin Pellets) | 8 |
| Isopropyl Palmitate | (IPP) | 3 |
| Octyldodecanol | (Eutanol G) | 3 |
| Persea Gratissima | (Avocadoöl) | 2,5 |
| Glyceryl Stearate | (Tegin 4100 Pellets) | 3 |
| Cetyl Alkohol | (Lanette 16) | 2,5 |
| Cyclomethicone | (Belsil CM 040) | 0,5 |
| Dimethicone | (Belsil DM 350) | 0,5 |
| Myristil Myristate | (Cetiol MM) | 0,3 |
| Algenextrakt | (Vegetol Algues) | 0,3 |
| Citronelyl Methylcrotonate | (Sinodor) | 0,3 |
| Allantoin | | 0,2 |
| BHT | | 0,15 |
| Xanthan Gum | (Kezan ST) | 0,25 |
| Ethylparaben | (Dekaben MEP) | 0,6 |
| Methylparaben | | |
| Phenoxyethanol | | |
| Propylparaben | | |
| Parfum | | 0,5 |
| Wasser | | 53,4 |

### BEISPIELE

### Beispiel 1

Die Schaumcremes werden in einer heiz- und kühlbaren geschlossenen Apparatur mit einem selbstaustragenden Homogenisator und einem heizbaren Dosiertrichter hergestellt.

Die Herstellung der Phase I erfolgt in einem beheizbaren Dosiertrichter durch Aufschmelzen einer Mischung enthaltend 2 Gew.-% Glycerylstearat, 4 Gew.-% Cetarylalkohol, 5 Gew.-% Stearinsäure, 1 Gew.-% Paraffin, 1 Gew.-% Tricetearet-4-phosphat bei 75° C. Es erfolgt eine dosierte Zugabe dieser Phase unter Rühren zu einer in der heiz- und kühlbaren geschlossenen Apparatur mit einem selbstaustragenden Homogenisator vorgelegten Phase II. Diese Phase besteht aus einer wässrigen Mischung enthaltend 2,5 Gew.-% Propylenglycol, 2,5 Gew.-% Glycerin, 2 Gew.-% Natriumlaurylsarcosinat sowie 0,3 Gew.% Allantoin und 10 Gew.-% Harnstoff sowie 0,1 Gew.-% Hyaluronsäure mit einem Molekulargewicht von mindestens 1.5 x 10⁶ Da. Die Menge an Wasser beträgt 69,6 Gew.-%. Es wird eine homogene Vermischung der Phasen I und II eingestellt.

Die dosierte Zugabe der Phase I erfolgt bei einer Temperatur von 75° C. Beide Phasen werden unter ständiger mittlerer Rührgeschwindigkeit zusammengeführt, wobei auf eine gleichmäßige Homogenisierungstätigkeit geachtet werden muss. Die Temperatur wird zwischen 5 und 20 Minuten bei 75° C gehalten. Die erhaltene Mischung/Emulsion wird unter ständigem Rühren auf eine Temperatur zwischen 30° und 40° C abgekühlt.

Nach Erreichen einer Temperatur von 40° C können dann auch weitere Stoffe zugesetzt werden. Dabei kann auch der pH-Wert auf einen Wert zwischen 5,5 und 8 oder 6,5 und 7,5 eingestellt werden. Hierzu wird 2-Amino-2-methyl-1-propanol oder KOH, bevorzugt jedoch KOH, eingesetzt. Es wird für eine hinreichend lange Zeit bis zur Stabilisierung des pH-Wertes gerührt und danach die Abfüllung in geeignete Lagerbehälter bzw. in entsprechende Sprühbehälter durchgeführt. Dabei werden 91 Gew.-%. Emulsion mit 9 Gew.-% Butan/Propan abgefüllt.

Weitere beispielhafte Schaumcreme-Zusammensetzungen, die gemäß dem Verfahren von Beispiel 1 hergestellt werden können, sind in den folgenden Beispielen 2 bis 7 angegeben.

### Beispiel 2

5,5 % Harnstoff und 0,2 % Hyaluronsäure

| Rohstoff | Handelsbezeichnung | g/kg | Gew.-% |
|---|---|---|---|
| Decyl Oleat | (Cetiol V) | 50 | 5 |
| Octyldodecanol | (Eutanol G) | 50 | 5 |
| Dimethicone | (Dow Corning | 2 | 0,2 |
| | Fluid 200/350 cst) | | |
| Glyceryl Stearat | (Cutina MD) | 20 | 2 |
| Cetearyl Alkohol | (Lanette O) | 40 | 4 |
| Stearinsäure | (Edenor C 18 98/100) | 50 | 5 |
| Hyaluronsäure | | 2 | 0,2 |
| hochmolekular | | | |
| Tristearat-4-phosphat | (Hostaphat KW 340 D) | 10 | 1 |
| Wasser demin. | | 649,4 | 64,94 |
| Harnstoff | | 55 | 5,5 |
| (kosm. Qualität) | | | |
| Propylenglykol 1,2 | | 25 | 2,5 |
| Glycerin 86 %ig | | 25 | 2,5 |
| Sodium Lauroyl | (Protelan LS 9011) | 20,0000 | 2 |
| Sarcosinat | | | |
| Allantoin | | 1 | 0,1 |
| KOH | | 0,6 | 0,06 |
| | | | |
| | Gesamt | 1000 | 100 |

### Beispiel 3

11 % Harnstoff und 0,2 % Hyaluronsäure

| Rohstoff | (Handelsbezeichnung) | g/kg | Gew.-% |
|---|---|---|---|
| Decyl Oleat | (Cetiol V) | 75 | 7,5 |
| Octyldodecanol | (Eutanol G) | 75 | 7,5 |
| Dimethicone | (Dow Corning | 2 | 0,2 |
| | Fluid 200/350 cst) | | |
| Glyceryl Stearat | (Cutina MD) | 20 | 2 |
| Cetearyl Alkohol | (Lanette O) | 40 | 4 |
| Stearinsäure | (Edenor C 18 98/100) | 50 | 5 |
| Hyaluronsäure | | 2 | 0,2 |
| hochmolekular | | | |
| Tristearat-4-phosphat | (Hostaphat KW 340 D) | 10 | 1 |
| demin. Wasser | | 544,5 | 54,45 |
| Harnstoff | | 110 | 11 |
| (kosm. Qualität) | | | |
| Propylenglykol 1,2 | | 25 | 2,5 |
| Glycerin 86 %ig | | 25 | 2,5 |
| Sodium Lauroyl | (Protelan LS 9011) | 20 | 2 |
| Sarcosinat | | | |
| Allantoin Pulver | | 1 | 1 |
| KOH | | 0,5 | 0,5 |
| | | | |
| | Gesamt | 1000 | 100 |

### Beispiel 4

11 % Harnstoff und 0,6 % Pentavitin

| Rohstoff | Handelsbezeichnung | g/kg | Gew.-% |
|---|---|---|---|
| Decyl Oleat | (Cetiol V) | 75 | 7,5 |
| Octyldodecanol | (Eutanol G) | 75 | 7,5 |
| Dimethicone | (Dow Corning | 2 | 0,2 |
| | Fluid 200/350 cst) | | |
| Glyceryl Stearat | (Cutina MD) | 20 | 2 |
| Cetearyl Alkohol | (Lanette O) | 40, | 4 |
| Stearinsäure | (Edenor C 18 98/100) | 50 | 5 |
| Kohlenhydrat-Gemisch | (Pentavitin) | 6 | 0,6 |
| Tristearat-4-phosphat | (Hostaphat KW 340 | 10 | 1 |
| | D) | | |
| Wasser demin. | | 540,5 | 54,05 |
| Harnstoff | | 110 | 11 |
| (kosm. Qualität) | | | |
| Propylenglykol 1,2 | | 25 | 2,5 |
| Glycerin 86 %ig | | 25 | 2,5 |
| Sodium Lauroyl | (Protelan LS 9011) | 20 | 2 |
| Sarcosinat | | | |
| Allantoin | | 1 | 0,1 |
| KOH | | 0,5 | 0,05 |
| | | | |
| | Gesamt | 1000 | 100 |

### Beispiel 5

11 % Harnstoff und 1,25 % Pentavitin

| Rohstoff | Handelsbezeichnung | g/kg | Gew.-% |
|---|---|---|---|
| Decyl Oleat | (Cetiol V) | 75 | 7,5 |
| Octyldodecanol | (Eutanol G) | 75 | 7,5 |
| Dimethicone | (Dow Corning Fluid 200/350 cst) | 2 | 0,2 |
| Glyceryl Stearat | (Cutina MD) | 20 | 2 |
| Cetearyl Alkohol | (Lanette O) | 40 | 4 |
| Stearinsäure | (Edenor C 18 98/100) | 50 | 5 |
| Kohlenhydrat-Gemisch | (Pentavitin) | 12,5 | 1,25 |
| Tristearat-4-phosphat | (Hostaphat KW 340 D) | 10 | 1 |
| Wasser demin. | | 534 | 53,4 |
| Harnstoff | | 110 | 11 |
| (kosm. Qualität) | | | |
| Propylenglykol 1,2 | | 25 | 2,5 |
| Glycerin 86 %ig | | 25 | 2,5 |
| Sodium Lauroyl | (Protelan LS 9011) | 20 | 2 |
| Sarcosinat | | | |
| Allantoin | | 1 | 0,1 |
| KOH | | 0,5 | 0,05 |
| | | | |
| | Gesamt | 1000 | 100 |

### Beispiel 6

16,5 % Harnstoff und 0,6 % Pentavitin und 0,1 % Portulak

| Rohstoff | Handelsbezeichnung | g/kg | Gew.-% |
|---|---|---|---|
| Decyl Oleat | (Cetiol V) | 75 | 7,5 |
| Octyldodecanol | (Eutanol G) | 75 | 7,5 |
| Dimethicone | (Dow Corning | 2 | 0,2 |
| | Fluid 200/350 cst) | | |
| Glyceryl Stearat | (Cutina MD) | 20 | 2 |
| Cetearyl Alkohol | (Lanette O) | 40 | 4 |
| Stearinsäure | (Edenor C 18 98/100) | 50 | 5 |
| Kohlenhydrat-Gemisch | (Pentavitin) | 6 | 0,6 |
| Tristearat-4-phosphat | (Hostaphat KW 340 D) | 10 | 1 |
| Wasser demin. | | 484,3 | 48,43 |
| Harnstoff | | 165 | 16,5 |
| (kosm. Qualität) | | | |
| Propylenglykol 1,2 | | 25 | 2,5 |
| Glycerin 86 %ig | | 25 | 2,5 |
| Sodium Lauroyl | (Protelan LS 9011) | 20 | 2 |
| Sarcosinat | | | |
| Allantoin | | 1 | 0,1 |
| KOH | | 0,7 | 0,07 |
| Portulak | | 1 | 0,1 |
| | | | |
| | Gesamt | 1000 | 100 |

### Beispiel 7

16,5 % Harnstoff und 1,25 % Pentavitin und 0,1 % Portulak

| Rohstoff | Handelsbezeichnung | g/kg | Gew.-% |
|---|---|---|---|
| Decyl Oleat | (Cetiol V) | 75 | 7,5 |
| Octyldodecanol | (Eutanol G) | 75 | 7,5 |
| Dimethicone | (Dow Corning | 2 | 0,2 |
| | Fluid 200/350 cst) | | |
| Glyceryl Stearat | (Cutina MD) | 20 | 2 |
| Cetearyl Alkohol | (Lanette O) | 40 | 4 |
| Stearinsäure | (Edenor C 18 98/100) | 50 | 5 |
| Kohlenhydrat-Gemisch | Pentavitin | 12,5 | 1,25 |
| Tristearat-4-phosphat | (Hostaphat KW 340 D) | 10 | 1 |
| Wasser demin. | | 477,8 | 47,78 |
| Harnstoff | | 165 | 16,5 |
| (kosm. Qualität) | | | |
| Propylenglykol 1,2 | | 25 | 2,5 |
| Glycerin 86 %ig | | 25 | 2,5 |
| Sodium Lauroyl | (Protelan LS 9011) | 20 | 2 |
| Sarcosinat | | | |
| Allantoin | | 1 | 0,1 |
| KOH | | 0,7 | 0,07 |
| Portulak | | 1 | 0,1 |
| | | | |
| | Gesamt | 1000 | 100 |

### Beispiel 8

### Tages- und Nachthautpflegeset

Tagespflegezusammensetzung: Schaumcremeformulierung mit einer Emulsion enthaltend 16,5 % Harnstoff und 1 % Nachtkerzenöl

| Rohstoff | Handelsbezeichnung | Menge/kg | Gew.-% |
|---|---|---|---|
| Decyl Oleat | (Cetiol V) | 7 | 7 |
| Octyldodecanol | (Eutanol G) | 7 | 7 |
| Dimethicone | (Dow Corning | 0,2 | 0,2 |
| | Fluid 200/350 cst) | | |
| Glyceryl Stearat | (Cutina MD) | 2 | 2 |
| Cetearyl Alkohol | (Lanette O) | 4 | 4 |
| Stearinsäure | (Edenor C 18 98/100) | 5 | 5 |
| Paraffinöl P-615 EL | | 1 | 1 |
| Tristearat-4-phosphat | (Hostaphat KW 340 D) | 1 | 1 |
| Wasser demin. | | 47,7 | 47,7 |
| Harnstoff | | 16,5 | 16,5 |
| Monopropylenglykol 1,2 | | 2,5 | 2,5 |
| Glycerin 86 %ig | | 2,5 | 2,5 |
| Sodium Lauroyl | (Protelan LS 9011) | 2 | 2 |
| Sarcosinat | | | |
| Allantoin | | 0,1 | 0,1 |
| Nachtkerzenöl | | 1 | 1 |
| AMP | | 0,5 | 0,5 |
| | | | |
| | Gesamt | 100 | 100 |

Nachtpflegezusammensetzung in Form einer Salbe mit folgender Zusammensetzung:

| Rohstoff | (Handelsbezeichnung) | Gew.-% |
|---|---|---|
| Harnstoff | | 15 |
| Glycerin | | 6 |
| Glyceryl Stearate SE | (Tegin Pellets) | 8 |
| Isopropyl Palmitate | (IPP) | 3 |
| Octyldodecanol | (Eutanol G) | 3 |
| Persea Gratissima | (Avocadoöl) | 2,5 |
| Glyceryl Stearate | (Tegin 4100 Pellets) | 3 |
| Cetyl Alkohol | (Lanette 16) | 2,5 |
| Cyclomethicone | (Belsil CM 040) | 0,5 |
| Dimethicone | (Belsil DM 350) | 0,5 |
| Myristil Myristate | (Cetiol MM) | 0,3 |
| Algenextrakt | (Vegetol Algues) | 0,3 |
| Citronelyl Methylcrotonate | (Sinodor) | 0,3 |
| Allantoin | | 0,2 |
| Xanthan Gum | (Kezan ST) | 0,25 |
| BHT | | 0,15 |
| Ethylparaben | | 0,6 |
| Methylparaben | | |
| Phenoxyethanol | | |
| Propylparaben | | |
| Parfum | | 0,5 |
| Wasser | | 53,4 |

### Beispiel 9

### Tages- und Nachtpflegeset

Tagespflegezusammensetzung gemäß Beispiel 2 und Nachtpflegezusammensetzung gemäß Beispiel 2 + 0,6 oder 1,25 % Pentavitin.

### Beispiel 10

Weitere, als Nachtpflegezusammensetzung einsetzbare Fettsalbe

| Rohstoff | (Handelsbezeichnung) | Gew.-% |
|---|---|---|
| Isopropylmyristat | (IPM) | 32,50 |
| Ethylhexyl Hydroxystearat, | | |
| Triethylhexyl Trimellitat, | | |
| C30-45 Olefin | (Clearwax) | 6,00 |
| Cetyl Ricinolat | (Tegosoft CR) | 2,00 |
| Helianthus Annuus | (Sonnenblumenöl) | 20,00 |
| Prunus Dulcis | (Mandelöl) | 16,00 |
| Citronellyl Methylcrotonat | (Sinodor) | 0,50 |
| Tocopheryl Acetat | | 3,00 |
| Cetearyl Isononanoat | (Tegosoft CI) | 20,00 |

### Beispiel 11

Weitere, als Nachtpflegezusammensetzung einsetzbare Salbe mit 10 % Harnstoff, 1 % Pentavitin und 0,2 % Natriumhyaluronat

| Rohstoffe | (Handelsbezeichnung) | Gew.-% |
|---|---|---|
| Harnstoff | | 10,00 |
| Glycerin | | 4,00 |
| Glyceryl Stearat | (Tegin M Pellets) | 5,00 |
| Isopropyl Palmitat | (Tegosoft P) | 3,00 |
| Octyldodecanol | (Tegosoft G 20) | 2,50 |
| Cera Alba | (Cera Alba) | 1,50 |
| Cetearyl Isononanoat | (Tegosoft CI) | 2,00 |
| Cetyl Alkohol | (Tego Alkanol 16) | 2,00 |
| Cyclomethicon | (Abil Wax 9840) | 0,50 |
| Dimethicon | (Belsil DM 350) | 0,30 |
| Isopropyl Myristat | (Tegosoft M) | 6,00 |
| Citronellyl | | |
| Methylcrotonat | (Sinodor) | 0,40 |
| Natrium Hyaluronat | (Hya Care) | 0,20 |
| Sorbitol | (Karion F) | 1,50 |
| Kohlenhydratgemisch | (Pentavitin) | 1,00 |
| Natrium Levulinat, | | |
| Natrium Anisat | (Dermosoft 1388) | 3,00 |
| Zitronensäure | | 0,10 |
| Tocopheryl Acetat | | 2,00 |
| Wasser | | 54,36 |

| | | |
|---|---|---|
| pH-Wert 5,0 - 5,2 mit Citronensäure einstellen | | |

### Beispiel 12

### Optischer Stabilitätstest

Die Formulierungen werden zwei Wochen bei ca. 11 °C oder bei ca. 35 °C gelagert und anschließend auf eine glatte Oberfläche aufgesprüht. Die Stabilität der versprühten Schäume wird optisch beurteilt und auf Basis einer Bewertungsskala von 1 - 4, wobei 1 die schlechteste Stabilität und 4 die beste Stabilität darstellt, bewertet.

Beispiele 1 (a - e) basieren auf der Zusammensetzung gemäß Beispiel 2 (5,5 % Harnstoff), Beispiele 2 (a - e) basieren auf der Zusammensetzung gemäß Beispiel 3 (11 % Harnstoff) und Beispiele 3 (a - e) basieren auf der Zusammensetzung von Beispiel 2 oder 3, wobei 16,5 % Harnstoff enthalten sind. Bei der jeweiligen Formulierung a - e wurde die Base und die Menge an Hyaluronsäure verändert und die Stabilität nach Lagerung getestet.

| Base | | | | Hyaluronsäure | | Stabilitätsbeurteilung nach Lagerung bei: | |
|---|---|---|---|---|---|---|---|
| Beispiel | AMP | NaOH | KOH | 0,1 % | 0,2 % | 11°C | 35 °C |
| 1a | x | | | | | 1 | 1 |
| 1b | | x | | | | 1 | 1 |
| 1c | | | x | | | 2 | 2 |
| 1d | | | x | x | | 2 | 4 |
| 1e | | | x | | x | 3 | 4 |
| 2a | x | | | | | 1 | 1 |
| 2b | | x | | | | 1 | 1 |
| 2c | | | x | | | 2 | 2 |
| 2d | | | x | x | | 3 | 4 |
| 2e | | | x | | x | 3 | 4 |
| 3a | x | | | | | 1 | 1 |
| 3b | | x | | | | 1 | 1 |
| 3c | | | x | | | 3 | 3 |
| 3d | | | x | x | | 1 | 4 |
| 3e | | | x | | x | 3 | 4 |

Man erkennt, dass die KOH-Beispiele stabiler sind als die AMP- oder NaOH-Beispiele. Man erkennt ferner, dass durch die Verwendung von Hyaluronsäure ein Stabilitätszuwachs bewirkt wird und durch die Verwendung von 0,2 Gew.-% anstelle von 0,1 Gew.-% ein weiterer Zuwachs an Stabilität verursacht wird. Die besten Ergebnisse werden bei Verwendung von KOH als Base und 0,2 % Hyaluronsäure erzielt.

Weitere bevorzugte Ausführungsformen der Erfindung sind wie folgt:
1. Formulierung, umfassend eine wässrige Emulsion und Treibgas, für eine Schaumhautcreme,
   **gekennzeichnet dadurch, dass** die Emulsion Harnstoff und Hyaluronsäure, mindestens eine freie Fettsäure und mindestens zwei Emulgatoren umfasst, wobei die Hyaluronsäure ein Molekulargewicht von mindestens 1 x 10⁵ Da
   aufweist.
2. Formulierung gemäß Gegenstand 1, wobei die Hyaluronsäure ein Molekulargewicht von mindestens 5 x 10⁵ Da aufweist.
3. Formulierung gemäß Gegenstand 2, wobei die Hyaluronsäure ein Molekulargewicht von mindestens 1 x 10⁶ Da aufweist.
4. Formulierung gemäß einem der Gegenstände 1 - 3, wobei die Emulsion
   3 - 30 Gew.-% Harnstoff und
   0,01 - 5 Gew.-% Hyaluronsäure
   umfasst.
5. Formulierung gemäß einem der Gegenstände 1 - 3, wobei die Emulsion
   5,5 - 25 Gew.-% Harnstoff und
   0,01 - 0,9 Gew.-% Hyaluronsäure
   umfasst.
6. Formulierung gemäß Gegenstand 5, wobei die Emulsion etwa 11 Gew.-% Harnstoff und etwa 0,2 Gew.-% Hyaluronsäure umfasst.
7. Formulierung, umfassend eine wässrige Emulsion und Treibgas, für eine Schaumhautcreme,
   **gekennzeichnet dadurch, dass** die Emulsion Harnstoff und ein Kohlenhydratgemisch, welches aus natürlich vorkommenden Kohlenhydraten gewonnen wird,
   mindestens eine freie Fettsäure und mindestens zwei Emulgatoren umfasst.
8. Formulierung gemäß Gegenstand 7, wobei das Kohlenhydratgemisch in seiner Zusammensetzung den in der Humanhaut enthaltenen Kohlenhydraten entspricht.
9. Formulierung gemäß Gegenstand 8, wobei das Kohlenhydratgemisch in Form einer wässrigen Lösung mit 50 - 55 Gew.-% Trockenrückstand eingesetzt wird.
10. Formulierung gemäß Gegenstand 9, wobei die Emulsion
   3 - 30 Gew.-% Harnstoff und
   0,01 - 5 Gew.-% der wässrigen Lösung
   umfasst.
11. Formulierung gemäß Gegenstand 9, wobei die Emulsion
   5,5 - 25 Gew.-% Harnstoff und
   0,01 - 0,9 Gew.-% der wässrigen Lösung
   umfasst.
12. Formulierung gemäß Gegenstand 11, wobei die Emulsion etwa 11 Gew.-% Harnstoff und etwa 0,6 Gew.-% der wässrigen Lösung umfasst.
13. Formulierung gemäß Gegenstand 11, wobei die Emulsion etwa 11 Gew.-% Harnstoff und etwa 1,25 Gew.-% der wässrigen Lösung enthält.
14. Formulierung gemäß einem der Gegenstände 1 - 13, wobei die Emulsion Harnstoff, Hyaluronsäure und ein Kohlenhydratgemisch, welches aus natürlich vorkommenden Kohlenhydraten gewonnen wird und welches in seiner Zusammensetzung den in der Humanhaut enthaltenen Kohlenhydraten entspricht, umfasst.
15. Formulierung gemäß Gegenstand 14, wobei die Emulsion
   5,5 - 25 Gew.-% Harnstoff,
   0,01 - 0,9 Gew.-% Hyaluronsäure und
   0,01 - 2 Gew.-% des Kohlenhydratgemisches, in Form einer wässrigen
   Lösung mit etwa 50 - 55 Gew.-% Trockenrückstand umfasst.
16. Formulierung gemäß Gegenstand 15, wobei die Emulsion
   5,5 - 25 Gew.-% Harnstoff,
   etwa 0,2 Gew.-% Hyaluronsäure und
   etwa 0,6 Gew.-% oder etwa 1,25 Gew.-% des Kohlenhydratgemisches,
   umfasst.
17. Formulierung, umfassend eine wässrige Emulsion und Treibgas für eine Schaumhautcreme, **dadurch gekennzeichnet, dass** die Emulsion mindestens eine freie Fettsäure und mindestens zwei Emulgatoren umfasst und mit Hilfe von Kaliumhydroxid auf einen pH-Wert von 5,5 - 8 eingestellt ist.
18. Formulierung, gemäß Gegenstand 17, wobei die Emulsion auf einen pH-Wert von 6,5 - 7,5 eingestellt ist.
19. Formulierung gemäß einem der Gegenstände 17 und 18, ferner umfassend Harnstoff und Hyaluronsäure gemäß den Gegenständen 1 - 6.
20. Formulierung gemäß einem der Gegenstände 17 und 18, ferner umfassend Harnstoff und ein Kohlenhydratgemisch gemäß den Gegenständen 7 - 13.
21. Formulierung gemäß einem der Gegenstände 17 und 18, ferner umfassend Harnstoff, ein Kohlenhydratgemisch und Hyaluronsäure gemäß den Gegenständen 14 - 16.
22. Formulierung gemäß einem der Gegenstände 19 - 21, wobei die Emulsion
   5,5 - 25 Gew.-% Harnstoff,
   etwa 0,2 Gew.-% Hyaluronsäure und
   optional etwa 0,6 Gew.-% oder 1,25 Gew.-% des Kohlenhydratgemisches in Form einer wässrigen Lösung mit etwa 50 - 55 Gew.-% Trockenrückstand umfasst und mit Hilfe von Kaliumhydroxid auf einen pH-Wert von 6,5 - 7,5 eingestellt ist.
23. Formulierung gemäß einem der voranstehenden Gegenstände 1 - 22, wobei die mindestens eine Fettsäure aus den C₁₂ - C₂₂ Fettsäuren ausgewählt ist.
24. Formulierung gemäß Gegenstand 23, wobei die mindestens eine freie Fettsäure aus den natürlichen C₁₂ - C₂₀-Fettsäuren, insbesondere Stearinsäure, Palmitinsäure und Myristinsäure, ausgewählt ist.
25. Formulierung gemäß einem der voranstehenden Gegenstände, wobei die Emulsion ferner ungesättigte und/oder mehrfach ungesättigte Fettsäuren umfasst.
26. Formulierung gemäß Gegenstand 25, wobei die Emulsion etwa 1 Gew.-% Nachtkerzenöl umfasst.
27. Formulierung gemäß Gegenstand 17 oder 18, wobei die Emulsion etwa 1 Gew.-% Nachtkerzenöl und etwas 16,5 Gew.-% Harnstoff umfasst.
28. Formulierung gemäß einem der voranstehenden Gegenstände, wobei die mindestens zwei Emulgatoren zum einen aus der Gruppe der nichtionischen Emulgatoren, insbesondere solche auf Fettalkohol-Basis und auf Basis von Partialestern von Fettsäuren, zum anderen aus der Gruppe der anionischen Emulgatoren, insbesondere der Sarkosinate, wie Lauryl-, Lauroyl- oder Cetylsarkosinat ausgewählt sind.
29. Formulierung gemäß einem der voranstehenden Gegenstände, wobei die Emulsion einen weiteren Coemulgator, ausgewählt aus der Gruppe der lipophilen Emulgatoren, wie Triceteareth-4-phosphat, Oleth-3-phosphat sowie lipophile Emulgatoren auf Basis niedrig ethoxylierter Fettalkohle umfasst.
30. Formulierung gemäß einem der voranstehenden Gegenstände, wobei die Emulsion ferner mindestens einen rückfettenden Stoff, ausgewählt aus der Gruppe bestehend aus Decyloleat, Isohexadecan, Stearinsäureglykolester, Kokosfettsäureethanolamid, Maisöl, Erdnussöl, Mandelöl, Sesamöl, Olivenöl, Jojobaöl, Sojaöl, Wollwachsalkohole, Paraffin, mittelkettige Triglyceride, Ölsäureoleylester, weißes Vaselin, Macrogol-Glycerolhydroxistearat, hydriertes Rizinusöl, Rizinusöl communis, Avocadoöl, Weizenkeimöl, Nachtkerzenöl, Sheabutter, Palmitinsäureisopropylester, Cetylpalmitat, Myristinsäuremyristilester und Octyldodecanol, umfasst.
31. Formulierung gemäß einem der voranstehenden Gegenstände, wobei die Emulsion frei von Paraffin ist.
32. Formulierung gemäß einem der voranstehenden Gegenstände, wobei die Emulsion ferner mindestens einen weiteren Bestandteil aus der Gruppe bestehend aus Moisturisern, Konservierungsmitteln, reizlindernden Substanzen, Hautpflegemitteln, hautwirksamen Vitaminen und siliziumhaltigen Substanzen wie Dimethicon umfasst.
33. Formulierung gemäß Gegenstand 32, wobei die Emulsion 0,01 - 2 %, bevorzugt 0,1 % Portulak umfasst.
34. Formulierung gemäß einem der voranstehenden Gegenstände, die einen pH-Wert von 5,5 - 8,0 aufweist.
35. Formulierung gemäß einem der voranstehenden Gegenstände, wobei die Emulsion
   1 - 10 Gew.-% Fettsäuren,
   5,5 - 25 Gew.-% Harnstoff,
   4 - 15 Gew.-% Emulgatoren,
   0,4 - 2,5 Gew.-% Coemulgatoren,
   1 - 10 Gew.-% Moisturiser,
   0,05 - 1 Gew.-% Hautpflegemittel, und
   0,5 - 6 Gew.-% rückfettende Stoffe sowie
   0,01 - 0,9 Gew.-% Hyaluronsäure und/oder 0,01 - 2 Gew.-% des
   Kohlenhydrat-Gemisches, umfasst.
36. Formulierung gemäß einem der voranstehenden Gegenstände, erhältlich durch
   - Herstellen einer Phase I durch Aufschmelzen bei 70° - 80° C einer Mischung, umfassend mindestens eine Fettsäure und mindestens einem nichtionischen Emulgator und mindestens einen Coemulgator,
   - gefolgt von einer dosierten Zugabe unter Rühren zu einer auf 70° - 80° C temperierten Phase II, umfassend Wasser, gegebenenfalls Harnstoff, Hyaluronsäure und/oder das Kohlenhydratgemisch, mindestens einen anionischen Emulgator, um eine homogene Mischung der Phase I und Phase II herzustellen,
   - Abkühlen der Mischung unter Rühren auf eine Temperatur von 30° - 40° C und
   - gegebenenfalls Einstellen des pH-Wertes und Abfüllen der erhaltenen Mischung in eine geeignete Darreichungsform unter Zugabe eines Treibgases.
37. Schaumhautcreme, erhältlich durch Versprühen einer Formulierung gemäß einem der voranstehenden Gegenstände.
38. Tages- und Nachthautpflegeset, umfassend eine Tageshautpflegezusammensetzung und eine Nachthautpflegezusammensetzung, wobei die Tageshautpflegezusammensetzung eine Schaumhautcreme ist, die mindestens eine freie Fettsäure und mindestens zwei Emulgatoren umfasst, wobei die Nachthautpflegezusammensetzung einen zusätzlichen Wirkstoff oder einen höheren Gew.-%-Anteil an Wirkstoff oder einen anderen Wirkstoff als die Tageshautpflegezusammensetzung umfasst, wobei der Wirkstoff aus der Gruppe der Lipidstoffe und der hydratisierenden Stoffe oder Mischungen davon ausgewählt ist.
39. Tages- und Nachthautpflegeset gemäß Gegenstand 38, wobei die Lipidstoffe gesättigte und ungesättigte Fettsäuren sowie die Gruppe der rückfettenden Stoffe gemäß Gegenstand 30 umfassen und die hydratisierenden Stoffe Harnstoff, hochmolekulare Hyaluronsäure, Kohlenhydratgemische, die aus natürlich vorkommenden Kohlenhydraten gewonnen werden und in ihrer Zusammensetzung den in der Humanhaut enthaltenen Kohlenhydraten entsprechen, und Sorbitol umfassen.
40. Tages- und Nachthautpflegeset gemäß Gegenständen 38 oder 39, wobei die Nachtpflegezusammensetzung einen zusätzlichen Wirkstoff, nämlich das Kohlenhydratgemisch in Form einer wässrigen Lösung mit etwa 50 - 55 Gew.-% Trockenrückstand umfasst.
41. Tages- und Nachthautpflegeset gemäß Gegenstand 38, wobei die Tageshautpflegezusammensetzung eine Formulierung gemäß den Gegenständen 1 - 6 ist und die Nachthautpflegezusammensetzung eine Formulierung gemäß den Gegenständen 14 - 16 ist.
42. Tages- und Nachthautpflegeset gemäß Gegenstand 38, wobei die Nachthautpflegezusammensetzung eine Salbe ist.
43. Tages- und Nachthautpflegeset gemäß Gegenstand 38, wobei die Tagespflegezusammensetzung eine Formulierung gemäß den Gegenständen 26 und 27 ist.
44. Verwendung einer Formulierung gemäß einem der Gegenstände 1 - 38 zur Behandlung von Dermatosen.
45. Verwendung eines Tages- und Nachtpflegesets gemäß einem der Gegenstände 38 - 43 zur Behandlung von Dermatosen.
46. Verfahren zur Behandlung von Dermatosen, bei welchen eine Formulierung gemäß einem der Gegenstände 1 - 37 auf den zu behandelnden Hautbereich aufgetragen wird.
47. Verfahren zur Behandlung von Dermatosen, bei welchem nach dem Aufstehen für die Tagespflege eine Tageshautpflegezusammensetzung und vor dem Schlafengehen für die Nachtpflege eine Nachthautpflegezusammensetzung gemäß den Gegenständen 38 bis 43 auf die zu behandelnde Haut aufgetragen wird.
48. Verwendung oder Verfahren gemäß einem der Gegenstände 44 - 47, bei welchem die Dermatosen , "trockenen Hautzustand", chronische Dermatosen, atopische Dermatitis, das allergische Kontaktekzem, das irritative Kontaktekzem, Psoriasis und das diabetische Fußsyndrom umfassen.

## Patentansprüche

1. Tages- und Nachthautpflegeset, umfassend eine
Tageshautpflegezusammensetzung und eine Nachthautpflegezusammensetzung, wobei die Tageshautpflegezusammensetzung eine Schaumhautcreme ist, die mindestens eine freie Fettsäure und mindestens zwei Emulgatoren umfasst, wobei die Nachthautpflegezusammensetzung einen zusätzlichen Wirkstoff oder einen höheren Gew.-%-Anteil an Wirkstoff oder einen anderen Wirkstoff als die Tageshautpflegezusammensetzung umfasst, wobei der Wirkstoff aus der Gruppe der Lipidstoffe und der hydratisierenden Stoffe oder Mischungen davon ausgewählt ist.

2. Tages- und Nachthautpflegeset gemäß Anspruch 1, wobei die Lipidstoffe gesättigte und ungesättigte Fettsäuren sowie die Gruppe der rückfettenden Stoffe, ausgewählt aus Decyloleat, Isohexadecan, Stearinsäureglykolester, Kokosfettsäureethanolamid, Maisöl, Erdnussöl, Mandelöl, Sesamöl, Olivenöl, Jojobaöl, Sojaöl, Wollwachsalkohole, Paraffin, mittelkettige Triglyceride, Ölsäureoleylester, weißes Vaselin, Macrogol-Glycerolhydroxistearat, hydriertes Rizinusöl, Rizinusöl communis, Avocadoöl, Weizenkeimöl, Nachtkerzenöl, Sheabutter, Palmitinsäureisopropylester, Cetylpalmitat, Myristinsäuremyristilester und Octyldodecanol, umfassen und die hydratisierenden Stoffe Harnstoff, hochmolekulare Hyaluronsäure, Kohlenhydrat-Gemische, die aus natürlich vorkommenden Kohlenhydraten gewonnen werden und in ihrer Zusammensetzung den in der Humanhaut enthaltenen Kohlenhydraten entsprechen, und Sorbitol umfassen.

3. Tages- und Nachthautpflegeset gemäß Anspruch 1 oder 2, wobei die Nachtpflegezusammensetzung einen zusätzlichen Wirkstoff, nämlich das Kohlenhydratgemisch in Form einer wässrigen Lösung mit etwa 50 - 55 Gew.-% Trockenrückstand umfasst.

4. Tages- und Nachthautpflegeset gemäß Anspruch 1, wobei die Tageshautpflegezusammensetzung eine Formulierung ist, umfassend eine wässrige Emulsion und Treibgas, für eine Schaumhautcreme, wobei die Emulsion Harnstoff und Hyaluronsäure, mindestens eine freie Fettsäure und mindestens zwei Emulgatoren umfasst, und wobei die Hyaluronsäure ein Molekulargewicht von mindestens 1 x 10⁵ Da aufweist, vorzugsweise mindestens 5 x 10⁵ Da, stärker bevorzugt mindestens 1 x 10⁶ Da; und die Nachthautpflegezusammensetzung eine Formulierung ist, umfassend Harnstoff, Hyaluronsäure und ein Kohlenhydratgemisch, welches aus natürlich vorkommenden Kohlenhydraten gewonnen wird und welches in seiner Zusammensetzung den in der Humanhaut enthaltenen Kohlenhydraten entspricht, und wobei die Emulsion vorzugsweise 5,5 bis 25 Gew.-% Harnstoff, 0,01 bis 0,9 Gew.-% Hyaluronsäure und 0,01 bis 2 Gew.-% des Kohlenhydratgemisches, in Form einer wässrigen Lösung mit etwa 50-55 Gew.-% Trockenrückstand umfasst, stärker bevorzugt 5,5 bis 25 Gew.-% Harnstoff, etwa 0,2 Gew.-% Hyaluronsäure und etwa 0,6 Gew.-% oder etwa 1,25 Gew.-% des Kohlenhydratgemisches umfasst.

5. Tages- und Nachthautpflegeset gemäß Anspruch 4, wobei die Tageshautpflegezusammensetzung eine Formulierung ist, welche 3-30 Gew.-% Harnstoff und 0,01-5 Gew.-% Hyaluronsäure, stärker bevorzugt 5,5-25 Gew.-% Harnstoff und 0,01-0,9 Gew.-% Hyaluronsäure, nock stärker bevorzugt etwa 11 Gew.-% Harnstoff und etwa 0,2 Gew.-% Hyaluronsäure umfasst.

6. Tages- und Nachthautpflegeset gemäß Anspruch 1, wobei die Nachthautpflegezusammensetzung eine Salbe ist.

7. Tages- und Nachthautpflegeset gemäß Anspruch 1, wobei die Tagespflegezusammensetzung eine Formulierung ist, welche ungesättigte und/oder mehrfach ungesättigte Fettsäuren, vorzugsweise aus Nachtkerzenöl oder Borretschöl, enthält.

8. Tages- und Nachtpflegeset gemäß einem der Ansprüche 1 bis 7 zur Verwendung bei der Behandlung von Dermatosen.

9. Tages- und Nachtpflegeset gemäß Anspruch 8, wobei nach dem Aufstehen für die Tagespflege eine Tageshautpflegezusammensetzung und vor dem Schlafengehen für die Nachtpflege eine Nachthautpflegezusammensetzung gemäß den Ansprüchen 1 bis 7 auf die zu behandelnde Haut aufgetragen wird.

10. Tages- und Nachtpflegeset gemäß einem der Ansprüche 8 oder 9, wobei die Dermatosen "trockenen Hautzustand", chronische Dermatosen, atopische Dermatitis, das allergische Kontaktekzem, das irritative Kontaktekzem, Psoriasis und das diabetische Fußsyndrom umfassen.
